# EUROPEAN PATENT SPECIFICATION

(11) **EP 2 083 919 B1**
(45) Date of publication and mention of the grant of the patent: **21.11.2012**
(21) Application number: 07827170.7
(22) Date of filing: 07.10.2007
(51) Int. Cl.: A61N 5/10

(54) **THULIUM-BASED CAPSULE AND DEVICES FOR USE IN HIGH DOSE RATE BRACHYTHERAPY**
KAPSEL AUF THULIUM-BASIS UND VORRICHTUNGEN ZUR VERWENDUNG IN DER HOCHDOSIERTEN BRACHYTHERAPIE
CAPSULE A BASE DE THULIUM ET DISPOSITIFS D'UTILISATION EN BRACHYTHERAPIE A DOSE ELEVEE

(30) Priority: 02.10.2006 US 848385 P
(43) Date of publication of application: 05.08.2009
(73) Proprietor: Shani, Gad, 84965 Omer (IL)
(72) Inventor: Shani, Gad, 84965 Omer (IL)
(74) Representative: Turner, Craig Robert
(86) International application number: PCT/IL2007/001196
(87) International publication number: WO 2008/041230

(56) References cited:
- EP-A- 1 232 770
- EP-A- 1 529 554
- EP-A1- 1 602 394
- WO-A-00/41185
- WO-A-99/40970
- US-A- 5 342 283

## Description

Brachytherapy is a cancer treatment method in which ionizing radiation source is inserted into, or adjacent, a cancerous tumor, in order to kill cancer cells or shrink tumors. There are two common Brachytherapy treatment types: i) High Dose Rate (HDR); and ii) Low Dose Rate (LDR), which are distinct from one another by the intensity of the radiation source, its form and its method of use.

In HDR brachytherapy, a high intensity (about 3 to 10 curie) radiation source is used and it is typically inserted into the tumor for a short period of time (between 10 minutes to a couple of hours). HDR brachytherapy is currently applied in treatments of breast, cervix, uterus, lung, eye and other kinds of cancerous tumors. The fact that the patient is hospitalized for few hours or for a day for HDR brachytherapy, and then released from the hospital clean of radioactivity, is very appealing.

In HDR brachytherapy the radiation source is generally stored in a lead shielding, and attached to one end of a flexible steel wire. The other end of the wire is attached to a system used for introducing the radiation source into the required irradiation location in the body of the treated subject, typically through a guiding catheter. The positioning of the radiation source in HDR brachytherapy is more accurate than in LDR brachytherapy, wherein the implanted radiation sources can migrate during the time period in which the radiation sources are in the body of the treated subject. Therefore, in HDR the irradiation time is very well controlled, and the dose of radiation delivered to the patient and the distribution of the radiation in the body of the patient is relatively accurate.

For the aforementioned reasons, HDR brachytherapy is becoming a popular treatment. The isotope which is currently mainly used in HDR brachytherapy is Ir192. Ir192 emits a large number of gamma rays photons having energy levels in the range of 66 keV (x-rays) to 885 keV. The highest intensity in the Ir192 photon spectrum is at the energy level of 317 keV, the next intense energy level is 468 keV. Photons emitted in these energy levels are highly penetrating in tissue and reach much beyond the tumor limits.

The HDR device described in US 2006/135841 comprises a safe for storing a capsule comprising a radioactive source and a transport wire wound onto a reel and connected to the capsule for delivering it via a transport tube to an applicator which has been introduced into a tissue to be irradiated. The radiation source in this device may be prepared from Ytterbium-169, Iodine-125, Palladium-103, Thulium-170 or Tungsten-181.

A radioactive radiation source is described in US Patent No. 5,282,781, wherein the radiation source is composed of a cylindrical tube having in a proximal section thereof a backbone wire for enhancing the flexibility of the tube and a cylindrical Ir-192 radioactive source placed adjacent to said backbone wire, wherein the cylindrical tube is sealed by a cylindrical plug, and wherein the external surface of the cylindrical tube is plated with gold (or other non-oxidizing material) throughout its length to preclude oxidation of the plated surface and to improve the sealing of the tip.

EP 1529 554A1 describes a radioactive radiation source for ophthalmic brachytherapy. The exemplified embodiment is based on a wire comprising an oxide of yttrium-90 dispersed within an aluminium matrix.

A flexible radiation source is described in US Patent No. 6,442,822, said radiation source, which is capable of being maneuvered through various body passages, comprise a thin cylindrical flexible tube having a modified segment at its proximal end and an unmodified section at its distal end, said sections are separated by a sealing plug, wherein the modified section contains a radioactive core made of various isotopes, and its wall thickness is thinner than the wall thickness of the unmodified section. The radiation source is optionally coated with a non-oxidizing agent, such as gold.

The inventor has now found that a beta-gamma radiation source, and more specifically, the isotope thulium 170 (Tm-170), may be effectively used as a radiation source for high dose rate brachytherapy, in combination with a radiation emission modifying metal, which is preferably selected from the group consisting of gold (Au) and platinum (Pt). It has been found that the radiation emitted from thulium 170, which is composed of beta radiation (electrons) and gamma radiation (photons), may be favorably modified by coating the thulium 170 with one or more of the heavy metals listed above, and more specifically with gold. By appropriately adjusting the thickness of the metal coating onto the thulium, it is possible control the intensities of the beta and gamma radiations emitted therefrom, generating a Tm-170 based high dose rate brachytherapy radiation source, characterized in that:
(i) the level of beta radiation emitted therefrom is reduced in comparison with that generated by Tm-170;
(ii) the photon emission spectrum of said source displays a lower ratio between the intensities of the characteristic peaks at 84 keV and 52 keV energy levels, relative to the ratio observed in the corresponding spectrum of Tm-170;
(iii) the photon emission spectrum of said source exhibits one or more peaks in the region between 65 keV and 88 keV energy levels indicative of photons emitted by the layer(s) of the radiation emission modifying metal;
(iv) the photon emission spectrum of said source comprises in addition to the discrete peaks also a continuous region indicative of the emission of braking energy associated with the layer(s) of the radiation emission modifying metal.

Photon spectrum measurements may be carried out using a conventional gamma spectrometry system, which includes: a gamma solid state detector, either HPGe or Si(Li), a high voltage supply, preamplifier, amplifier and a multychannel analyzer. Changes in the emission of beta radiation can be determined by applying a radiation detector, either a thin window ion chamber or a scintillator.

Accordingly, the present invention primarily relates to a capsule for high dose rate brachytherapy, wherein the capsule comprises within its interior space thulium-170, and further comprises at least one layer of a radiation emission modifying metal, wherein said layer is provided either internally within the capsule or on the outer surface thereof. The activity of the radiation source within the capsule is not less than 3 curie.

The term "capsule", as used herein, refers to a thin casing suitable for enclosing a radioactive source, which casing is deliverable to the tumor site by means of a guidewire via commonly applied applicators (e.g., catheters) in accordance with high dose rate brachytherapy protocols. Particularly preferred is a titanium capsule. Capsules of various structures may be used according to the present invention for incorporating the thulium therein, such as those described, for example, in US 6,179,768, US 6,196,964 and US 7,077,800. These references also describe combinations of a capsule and a guidewire, in order to allow the delivery of the capsule to the tumor site. The metal of which the capsule is made is of course distinct from the radiation emission modifying metal to be applied in accordance with the invention.

The thulium 170-containing capsule provided by the present invention preferably comprises a gold or a platinum coating which at least partially, and preferably entirely, surrounds the thulium 170, said coating being either interposed between the radioactive isotope and the walls of the capsule, or alternatively, applied onto the outer faces of the capsule. The capsule is capable of being delivered into a body site through a catheter, and has an activity in the range of 3-30 curie.

The capsule described above may be produced, for example, by coating thulium 169 with one or more layers of a radiation emission modifying metal (gold), placing the coated thulium 169 in a capsule, e.g., titanium capsule, sealing the capsule and submitting the same to a neutron activation in order to convert the thulium 169 into thulium 170. Alternatively, the coating is applied onto the capsule's outer faces. It is noted that thulium 169 to be converted according to the present invention to the radioactive thulium 170 is available in the form of a cylindrical body, namely, a wire of small diameter. This simplifies the preparation of the capsule and devices of the invention, as the shape of commercially available thulium 169 readily matches into the capsule.

In another aspect, the present invention relates to a high dose rate brachytherapy device, comprising:
a) a thulium-170 radioactive section attached to one end of a guiding wire, said radioactive section and a portion of said guiding wire being placed within the interior of a shell and affixed to the walls thereof, with the proximal end of the guiding wire extending beyond the end of said shell; and
b) a radiation emission modifying metal, provided either internally within the shell or on the outer surface thereof.

Preferably, the radiation emission modifying metal, and more specifically gold, is provided as a thin coating onto the surface of said radioactive section, such that a gold layer is interposed between said radioactive section and said shell. The thickness of the gold layer may be up to 0.15 mm. Alternatively, a gold layer is externally applied onto the shell.

The distal end of the device, to be positioned in proximate with the body site to be irradiated, is defined by said shell and the thulium isotope placed therein, attached to one end of the guiding wire; The proximal end of the device is defined by the second end of said guiding wire to be connected to a suitable machinery, as will be described in more detail below.

More specifically, the device has an essentially cylindrical symmetry. The shell may be provided in the form of a cylindrical surface having one open base. The thulium 170 isotope, in the form of a cylindrical body, is confined within said shell, with the guiding cable extending outwardly through the open base of said shell. Alternatively, the shell may be a tube, e.g., commercially available titanium tube, such that its distal end needs to be sealed following the insertion of the isotope therein.

In another aspect, the present invention provides a high dose rate brachytherapy method, which comprises placing proximate to the tumor site a radiation source comprising thulium 170 provided with a coating of a radiation emission modifying metal, which metal is preferably gold, wherein the activity of said radiation source is not less than 3 curie, and delivering radiation dose to said tumor for a period of time of about 10 minutes to a couple of hours, typically not more than three hours. A typical radiation dose can be in the range between 30 and 150 Gy (Gray).

### Brief Description of the Drawings

The present invention is illustrated by way of example in the accompanying drawings, in which similar references consistently indicate similar elements and in which:
- Fig. 1 illustrates a longitudinal-section view of a general structure of a HDR brachytherapy device of the invention;
- Fig. 2 shows a longitudinal-section view of a HDR brachytherapy device of the invention wherein the radioactive section is enclosed in a tube;
- Fig. 3 shows a longitudinal-section view of a brachytherapy device of the invention, wherein the heavy metal coating is applied externally;
- Fig. 4 shows a longitudinal section view of a capsule of the invention encased in a tubular body;
- Fig. 5 shows the characteristic photon spectrum emitted by Tm-170;
- Fig. 6 shows a photon energy spectrum measured for a Tm170 radiation source of the invention having a 0.1mm gold layer coating;
- Fig. 7 shows a photon spectrum of a Tm170 radiation source in which the radiating core was encased in a titanium capsule having wall thickness of about 0.05mm; and
- Fig. 8 schematically illustrates a delivery system for introducing a radiation source into an irradiation site in a treated subject.

It should be noted that the embodiments exemplified in the Figures are not intended to be in scale and are in diagram form to facilitate ease of understanding and description.

### Detailed Description of Preferred Embodiments

The present invention provides a radiation source, which may be used for high dose rate brachytherapy. The beta-gamma radiation source of the invention comprises the isotope thulium 170 (Tm170), wherein a preferable ratio between the beta and the gamma radiations emitted from said isotope is obtained by coating the radiation source by one or more layers made of metals that are most preferably selected from the group consisting of gold, platinum and lead; gold and platinum are especially preferred with gold being most preferred.

The Tm170 radiation source is preferably obtained by neutron activation of natural thulium - Tm169. The activated Tm170 emits both beta and gamma rays and therefore it can be used for treatment of small tumors as well as large tumors. The beta rays may be used for treating small tumors, while the gamma photons are more suitable for treating large tumors.

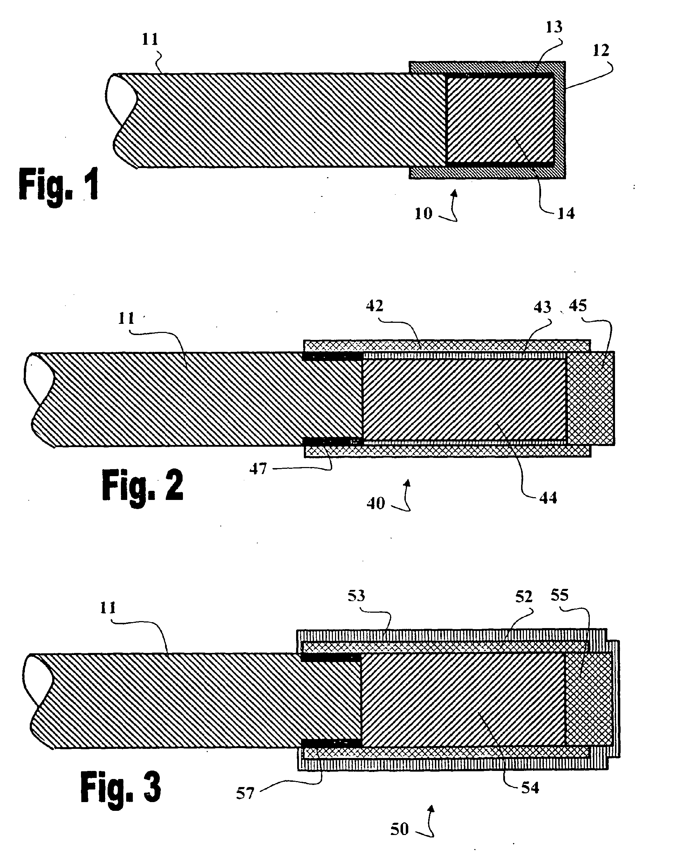

With reference to Fig. 1, a brachytherapy device **10** of the invention comprises a radioactive section **14** made of thulium 170, and having a heavy metal (e.g., Au or Pt ) coating **13** surrounding the same (the radioactive isotope is produced by activation of thulium 169 under conditions that are described hereinafter). The radioactive Tm170 section **14** is preferably a cylindrical body having a diameter generally in the range of 0.6 to 1.0 mm, preferably about 1 mm (or a little smaller), and having a length which corresponds to the desired activity. For example, possible lengths of core **14** are 1.5, 3 or 5 mm, for providing activities of 4 curie, 8 curie, or 15 to 30 curie, respectively. These preferable lengths give volumes of about 1.1775, 2.355 and 3.925 mm³, respectively, and weight of 10.95, 21.98 and 36.5 mg, respectively. The number of Tm 169 atoms in such volumes is of importance since the number of Tm170 atoms produced by the neutron activation is relative to the number of Tm169 atoms being activated. The numbers of activated Tm169 atoms are about: 3.89x10²², 7.78x10²² and 1.29x10²³, respectively. The number of Tm170 atoms obtained in the neutron activation equals to the product of the number of Tm169 atoms irradiated by the neutron fluence multiplied by the activation cross-section. (Neutron fluence =neutron flux times activation time). The activity (number of disintegrations per second) of the Tm170 core **14** is given by the number of Tm170 atoms times the decay constant.

The Tm 170 **14** is preferably contained in a cap **12** having an outer diameter of about 1.1mm and wall thickness of about 0.05mm. A thin wall is important for the utilization of the beta rays. A thicker wall will absorb a certain percent of the beta rays. The length of cap **12** depends on the length of the Tm section **14**. In a preferred embodiment of the invention, cap **12** is made from a type of inert metal, such as, but not limited to titanium or stainless steel, preferably from titanium, and the length of cap **12** is preferably about 1mm longer than the length of core **14** i.e. 2.5, 4 or 6 mm long. The device is prepared as follows. A piece of thulium 169, coated with gold is inserted into the cap **12**. Gold coating may be accomplished by numerous conventional methods. For example, electroplating may be applied, wherein the thulium is immersed in a gold solution and an electric current is passed through the solution to form a gold coating onto the object. A second method is by gold evaporation. Heat is applied to a gold wire or rod (generally by electric current). Due to the high temperature gold atoms are evaporated and deposited on the thulium. A third way is to wrap the thulium with a gold foil of the desired thickness. Thin gold foils of various thicknesses are commercially available (Goodfellow, England). The thulium section **14** may be attached to cap **12**, for example, by glue or by being snugly pressed thereinto.

The titanium cap **12** with the natural thulium (Tm169 - before activation) coated by the one or more heavy metal layers **13** contained thereinside, is transferred for neutron activation at a nuclear reactor.

Preferably, the thulium is activated by a neutron flux in the order of 10¹⁵n/cm²s, such that the activation time required is relatively short (e.g., 7-15 days). The activation time should be sufficient to obtain the desired activity, as explained above.

After obtaining the required activity, a guiding wire **11** is inserted into cap **12.** Guiding wire **11** is preferably made from a stiff and flexible material, such as, for example, steel. The length of wire **11** may generally be in the range of 200 to 1200 mm, preferably about 300 mm, and its diameter may generally be in the range of 0.7 to 1.0 mm, preferably about 1.0 mm.

The attachment of guiding wire **11** (to the titanium cap **12** comprising the thulium core **14** coated by gold layer **13**) is preferably carried out by means of an epoxy glue under pressure (the cap volume between the thulium and the steel rod is filled with epoxy). The diameter of guiding wire **11** is preferably slightly smaller than the inner diameter of cap **12**, such that a distal end portion of guiding wire **11** may be snugly introduced thereinto until it abuts core **14**. In this way, wire **11** may be pressed into cap **12**, and after the epoxy glue is dried a well-sealed strong connection is obtained.

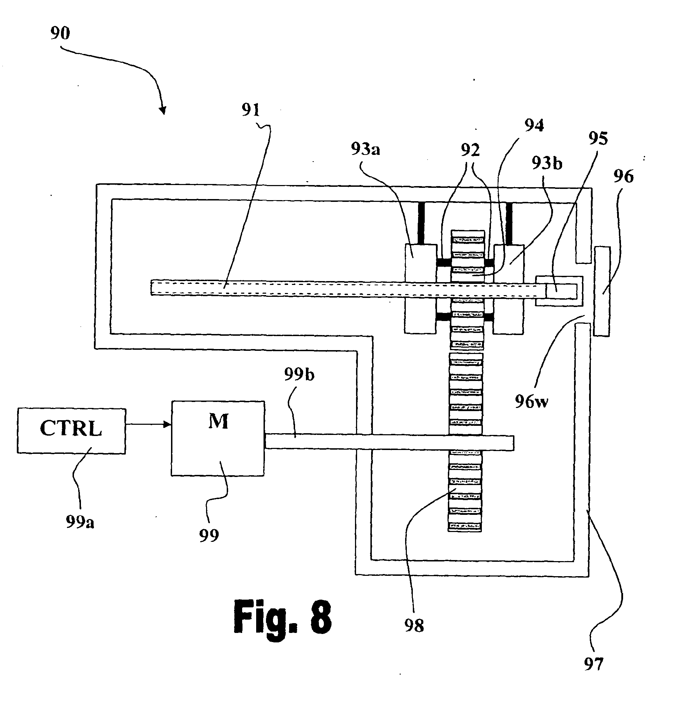

The device **10** with the guiding wire **11** is preferably kept in an appropriate lead shield (e.g., **97** in Fig. 8), and delivered through a properly placed guiding catheter towards the treatment site (cancerous tumor) within the body of the treated subject, by means of a remotely operated mechanism (possible delivery system is described hereinbelow with reference to Fig. 8). The device **10** is maintained in the treatment site within said guiding catheter (in the tumor) for a predetermined time (e.g., ranging between 10 minutes to few hours), sufficient to deliver the required dose of radiation (e.g., 30-150 Gy). The selected dosage regimen will depend on the severity of the condition being treated and other factors associated with the patient being treated.

Fig. 2 shows a longitudinal-section view of another preferred embodiment of a device **40** of the invention, wherein the radiating core **44** is enclosed in a tube **42.** The production of radiation source **40** may be substantially similar to that of device **10** shown in Fig. 1. Similarly, a cylindrical Tm core (Tm wire) is covered by one or more layers of heavy metal coatings **43** (e.g., Au, Pt or Pb), and it is then snugly introduced into tube **42** via one of its openings. The heavy metal coatings **43** may be applied by means of conventional metal plating techniques, as described above, or alternatively, by wrapping the Tm core **44** by a heavy metal foil adhered thereto by means of epoxy glue, for example. The thickness of the heavy metal coatings is as described above.

The length of Tm core **44** may generally be in the range of 1.0 to 5.0 mm, preferably about 5 mm, and its diameter may generally be in the range of 0.6 to 1.0 mm, preferably about 0.6 mm. Tube **42** may be manufactured from a type of inert metal, such as, but not limited to, titanium or stainless steel, preferably from titanium. The length of tube **42** may generally be in the range of 2.5 to 8.0 mm, preferably about 5.0 mm, its outer diameter may generally in range of 0.8 to 1.2 mm, preferably about 0.8 mm, and its wall thickness may generally be in the range of 0.05 to 0.1 mm, preferably about 0.1 mm.

After placing the Tm core **44** in tube **42**, one of the openings of tube **42** is sealed by a plug **45**, said plug **45** is advanced into tube **42** until it abuts Tm core **44.** Plug **45** is preferably made from one of the materials indicated above for tube **42**, preferably from titanium, its length being about 0. 5 mm, and its diameter is adapted to snugly fit into tube **42**. After sealing tube **42** with plug **45** and following welding, the radiation source may be activated in a nuclear reactor by means of a neutron flux in the order of 10¹⁴-10¹⁵ n/cm²s. For considerations of safety, the tube is delivered within a quartz ampoule to the nuclear reactor.

A guiding wire **11** is then attached to form part of the device by means of glue **47** (e.g., epoxy), which is placed in the opening of tube **42**, and thereafter wire **11** is pressed thereinto until its distal end abuts core **44**. This preferred embodiment is particularly advantageous in implementations in which the different members of the radiation source can not be attached by means of welding.

Fig. 3 shows a longitudinal-section view of yet another preferred embodiment of a device **50** of the invention, wherein the heavy metal coating **53** is applied externally. In principle, the construction of radiation source **50** is substantially similar to the construction of device **40** show in Fig. 2. In this embodiment, however, the Tm core **54** is placed in a tube **52** without the heavy metal coating. One end opening of tube **52** is similarly sealed by a plug **55** which is snugly fitted thereinto until it abuts Tm core **54** and then welded, and after activation, the other end opening of tube **52** is sealed by means of glue **57** applied thereinside and by the distal end portion of wire **11**, which is introduced thereinto until its distal tip abuts Tm core **54**.

Tm core **54** is preferably cylindrical in shape, its diameter may generally be in the range of 0.6 to 1.0 mm, preferably about 0.6 mm, and its length may generally be in the range of 1.0 to 5.0 mm, preferably about 5 mm. After sealing the end openings of tube **52,** tube **52** is covered by one or more heavy metal layers **53** (e.g., Au or Pt). Heavy metal layers **53** may be applied by means of conventional metal coating techniques, as described above, or alternatively, by wrapping the titanium surface by a foil made of said heavy metal material, and possibly adhering it thereto by means of epoxy glue, for example. Heavy metal layers **53** are preferably made from gold, and their entire length may generally be in the range of 2.5 to 8.0 mm, preferably about 5.0 mm. Heavy metal layers **53** are preferably applied over the entire length of tube **52**, namely the entire surface of the tube and the outer surfaces of plug **55** are covered by said layers **53** i.e., the front face of the radiation source is also covered by the heavy metal layers. In this way the intensity of the beta rays emitted from the radiating core is substantially reduced, or stopped, in all directions.

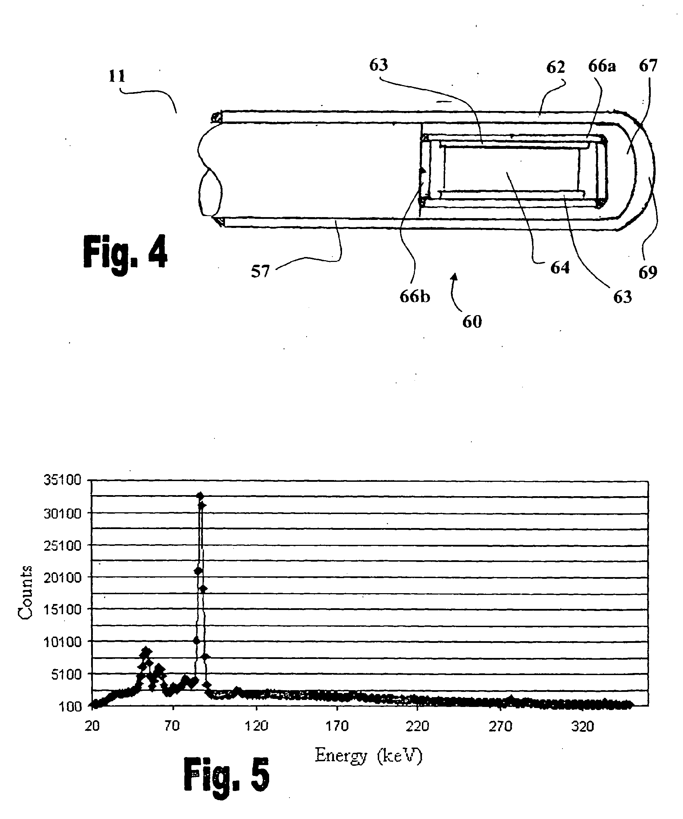

Fig. 4 illustrates a brachytherapy device used for sealing the capsule of the present invention. The device comprises a sealed, elongated tubular body having a distal portion and a proximal portion, wherein the capsule according to the invention is placed in said distal portion of said tubular body and is affixed to said position by means of a rod extending from said proximal portion of said tubular body to said capsule.

More specifically, Figure 4 shows a longitudinal section view of said device, wherein the capsule is positioned in its distal portion **67**. Tm core **64** is encased in an internal enclosure **66** (**66a**, **66b**), which defines the capsule walls. The capsule is prepared as follows. Tm core **64** is wrapped, or coated, by one or more gold or platinum layers **63** according to the techniques described before. The Tm core **64** with its coating **63** is then placed in a cylindrical enclosure **66**, said enclosure may be constructed from a tube **66a** made from titanium, for example. After placing the coated Tm core inside the tube **66a** its side openings are sealed by means of corresponding plugs or lids **66b**, made from the same material of tube **66a**, and welding the same thereover or thereinside, to form a capsule according to the present invention.

The thickness of coating layers **63** may generally be in the range of 0.01 to 0.15 mm, preferably about 0.1 mm. The length of Tm core **64** may generally be in the range of 1.0 to 5.0 mm, and its diameter may generally be in the range of 0.5 to 1.0 mm, preferably about 0.6 mm.

The titanium capsule **66** may be then placed in a cylindrical tube **57.** Tube **57** may be made of an inert metal, preferably of stainless steel, its length may generally be in the range of 10 to 200 mm, preferably about 100 mm, its outer diameter may generally be in the range of 1.0 to 1.3 mm, preferably about 1.2 mm, and its wall thickness may generally be in the range of 0.05 to 0.1 mm, preferably about 0.1 mm.

The distal end opening of cylindrical tube **57** is sealed by a weld **69** applied thereover such that a distal taper is assumed, and its proximal end is sealed by means of a rod **11** which extends through tube **57** from its proximal end and is contiguous with the titanium capsule, and welding the same therein. In this preferred embodiment of the invention it is assured that the radiating core is effectively sealed inside the radiation source, preferably by means welding, and is safely secured to its desired position in the device.

It is noted that the half-life of Tm170 is 128 days, which is substantially longer than the half-life of Ir192 (74 days). Therefore, the thulium-based source according to the present invention can serve for treating many patients during a long period before it has to be reactivated. Since the production of Tm170 is done by simple neutron activation, any source, which decays below the desired activity, can be reactivated in a neutron flux such that there is no need for a new radiation source whenever the activity of the radiation source substantially decays. The production simplicity of the Tm170 radiation source is a major advantage of the radiation source of the invention.

The radiation sources described above may be used in the treatment of both small and large tumors. The range of the 970 keV beta rays, which comprises 76% of the beta rays emitted from the Tm170, is distributed in the irradiated tissue within a radius of about 2 mm from core **14**. However, the presence of a metal (gold) coating of thickness of between few atomic layers (e.g., few microns) to about 0.15 mm favorably modifies the radiation emission profile of the source, in cases wherein the beta rays are not wanted or their intensity should be lowered. Specifically, in case of a thick heavy metal layer **13** (e.g., ~0.15mm), all beta radiation of energy of up to 1 MeV is blocked. The intensity of the photon radiation in this case is increased due to the bremssrahlung effect in the heavy metal coating layers **13** in which a part of the beta radiation turns into gamma photons. The radiation emission profiles of the modified source according to the present invention will now be discussed in more detail with reference to Figure 5 to 7. In these figures the abscissa is the energy of the emitted photon and the ordinate is the intensity, recorded by multychannel analyzer.

The radiation emitted from Tm 170 is composed of beta radiation (electrons) and gamma radiation (photons). 76% of the beta radiation is of energy Emax=968 keV and 24% of it is of energy 884 keV. Fig. 5 shows the characteristic photon spectrum of Tm 170 (non-coated). As seen in Fig. 5, the photon spectrum has a main peak at 84 keV energy level and another smaller peak, at energy 52 keV. More specifically, there are two photon energies emitted from Tm170, the first is composed of gamma rays having a 84 keV energy level emitted from excited Y170 atom formed upon the beta decay of Tm170, and the second is x-ray at about 52 keV energy level, emitted following the rearrangement of the Y170 electrons, after the interaction of some of the photons emitted from the nucleus, with the atomic electrons. The ratio of gamma to x-ray emission intensity is typically about 3:1.

When the beta rays emitted from the Tm 170 enter the heavy metal layer (e.g., gold) covering the thulium, most of them are stopped by collision with the electrons of the heavy metal atoms. This is the main beta removal effect caused by the heavy metal. A small fraction, 2-3% as shown in table 1, undergo the effect known as braking radiation (Bremsstrahlung in German) due to deceleration of these electrons in the electric field of the heavy metal electrons or the heavy'metal nuclei. The energy of those beta rays is turned into photons of continuous spectrum, mostly in the lower energy range. The fraction of the beta ray turning into photons by braking radiation is proportional to the beta energy times the atomic number of the stopping material. The fraction of the beta intensity turning into photons and the photons energy obtained for each beta ray are shown in table 1 for three possible heavy metals: platinum, gold or lead. The columns marked by the % symbol in table 1 indicate the percent of the beta rays turned into photons in the braking radiation process, for each beta energy. The columns entitled by "Energy" give the photon energy obtained from the beta rays, for each beta energy. The columns entitled by "76%" and "24%" give the actual photon energy obtained, from each beta energy. The last column is the sum of the two, i.e. total photon energy obtained from the beta rays by braking radiation.

**TABLE 1 - Photon fractions added to the spectrum due to Braking Radiation**

| | | | Eₘₐₓ= 968 keV | | | Eₘₐₓ= 884 keV | | | |
|---|---|---|---|---|---|---|---|---|---|
| Metal | Atomic Number | Density gm/cm³ | % | Energy [keV] | 76% [keV] | % | Energy [keV] | 24% [keV] | Total energy Per Disinte gration |
| Pt | 78 | 21.45 | 2.64 | 25.6 | 19.44 | 2.41 | 21.33 | 5.12 | 24.56 keV |
| Au | 79 | 19.3 | 2.68 | 25.9 | 19.69 | 2.44 | 21.6 | 5.19 | 24.88 keV |
| Pb | 82 | 11.35 | 2.78 | 26.9 | 20.44 | 2.45 | 22.43 | 5.38 | 25.82 keV |

When the source (Tm 169 coated with one of the heavy metal layers: gold, platinum or lead in a titanium capsule), is activated by neutrons to get the radioactive isotope Tm 170, the coating material is activated too and a radioactive isotope is formed. The activation of the titanium is negligible. These radioactive isotopes are short lived: T½Pt195=4 days, T½Au198=65 hours, T½Pb209=3.3 hours. The respective activation cross sections are: σPt=10 b, σAu=98.8 b and σPb=0.17 b. The activity of these isotopes is undesired in the radiation source, therefore, after being activated the radiation source should stay in the reactor area for about two weeks cooling period for these isotopes to decay. The two weeks decay time has a minor effect on the Tm 170 activity; it decays to 93% of its activity at the end of the activation.

The purpose of the heavy metal (Au or Pt) coating the thulium core in the radiation source of the present invention is to be a part of the radiation source and it is applied directly thereon. Although the heavy metal coating is not radioactive, it emits radiation as a result of absorbing the radiation from the Tm 170 core. The heavy metal coating applied over the radiation source reduces, or stops completely (depends on its thickness), the beta rays emitted from the Tm 170 core, from leaving the radiation source into the tumor. A part of the beta rays absorbed by the heavy metal coating the Tm turns into photons as braking radiation. An additional photon source is created due to x-ray emitted from the heavy metal coating, this x rays emission is typical to those metals. These x rays energies are in the range of 66-84 keV respective to the metal emitting it.

Thus, the metal (Au or Pt) coating the Tm 170 core of the radiation source changes the radiation emitted therefrom in a number of ways. The effect it has on the beta rays is the most drastic because this thin coating either eliminates the beta rays emission completely or reduces it to a desirable degree. The thicker the coating layer the weaker is the beta intensity.

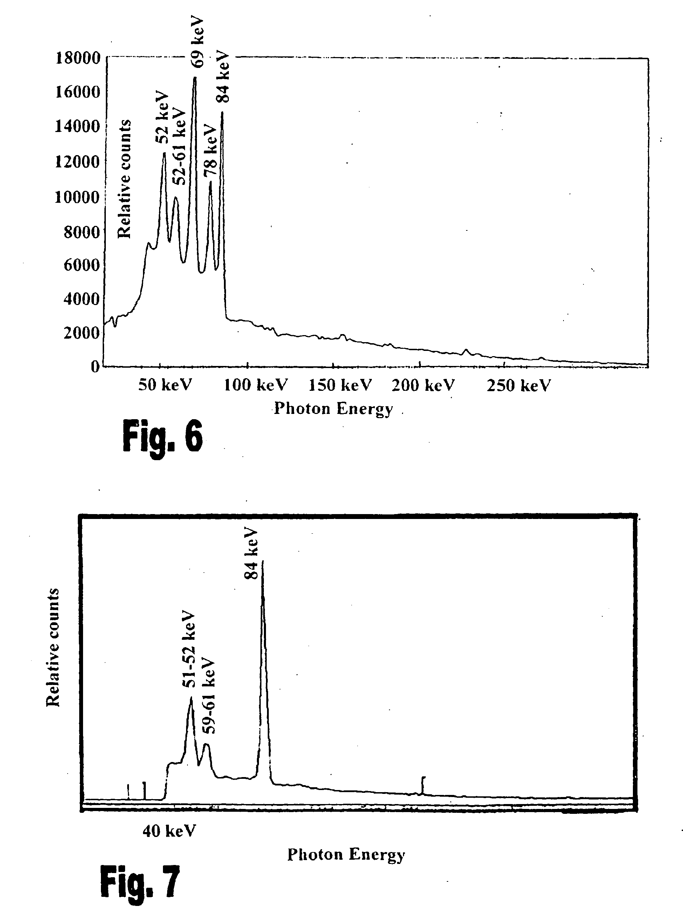

The second effect of the heavy metal coasting layer on the radiation emitted from the radiation source is the addition of a continuous x ray spectrum, to the photon emitted from the radiation source. Stopping the beta radiation by the heavy metal causes the emission of braking radiation. This is a continuous photon spectrum of energy from very low (1-3 keV) to the highest energy of the beta rays-Emax emitted from the Tm 170, as can be seen in figure 6.

The third effect of the heavy metal coating on the photon spectrum emitted from the radiation source is the additional peaks in the spectrum, related to x rays emitted from the metal. The source of these x rays is the photoelectric effect caused by the 84 keV photons emitted from the Tm 170. The main x ray peaks are at energie levels: 67, 69 , 78 and 80.1 for gold, 65, 67, 75.7 and 77.8 for platinum and 72.8, 75, 84.8 and 87.3 for lead. The main gold peaks are seen in figure 6. The photoelectric effect caused by the 84 keV photons lowers this peak in the spectrum as can be seen in figure 6. (The ratio between the 84 keV peak and the 52.4 keV peak has been reduced compared to that seen in Fig. 5; according to the modified source of the present invention, the ratio between said peaks is less than 2.5:1). This is fourth spectrum change due to the heavy metal coating the TM 170.

To sum up, the changes in the photon spectrum caused by the heavy metal layer are: additional low energy photons, reduction in the 84 keV peak and addition of x rays peaks corresponding to the metal coating the thulium radiation source. The change in the beta spectrum: lowering its intensity or complete removal.

The addition of the low energy photons to the spectrum increases the efficiency of the radiation source at small distance. Although lowering the 84 keV photons peak reduces the number of photons that can reach large distance from the source, this is sufficiently compensated by the addition of the higher energy x rays emitted by the heavy metal and by the higher photons in the braking radiation.

Fig. 7 (comparative) shows the photon spectrum of a Tm170 radiation source in which the radioactive section was not coated by gold but encased in a titanium capsule having wall thickness of about 0.05mm. The continuous spectrum beyond the 84 keV peak is due to braking radiation in the titanium.

The radiation source of the invention may be introduced into the body of the treated subject by employing conventional HDR procedures. For example, in use, the doctor inserts a catheter such as a hollow plastic tube into the patient's body. A mechanical computer-controlled delivery system called an afterloader is then used to remotely remove the radioactive source from the safe and move it through the catheter to the tumor site. The remote afterloading technique reduces the radiological risk to medical personnel of exposure to the high-activity source. A suitable procedure is also described in US 6,607,478. Commercial HDR brachytherapy systems are available from Varian and Nucleotron.

Figure 8 schematically illustrates a delivery system **90** designed for remotely advancing a radiation source into a treated subject. The radiation source **95** is stored in a lead box **97** having wall thickness about 1 cm. Lead box **97** houses the delivery mechanism, which is comprised of a stepping motor **99** (external to lead box **97**) driven by a controller **99a**, a gear **98**, a driving nut **94**, bearings **93a** and **93b**, and the threaded rod **91** holding the radiation source **95**. The motor **99** turns a gear wheel **98,** turning a nut **94**, which in turn drives the rod **91** with the radiation source **95** forward or backward.

Rod **91** is threaded along most of its length and it passes through a threaded passage provided in nut **94**. Strainers **92** keep the nut-gear **94** from moving along the rod as it turns. The bearings **93a** and **93b** are tight enough to keep the rod **91** from turning when the nut **94** is turning, but not too tight so that the rod can move back and forth.

When the radiation source is moved forward into the patient or back into the shielded box, corresponding control signals received from controller **99a** (electronic pulses received from a pulse generator or a suitable control logic may be employed) activates stepping motor **99** to rotate the nut **94** the respective number of turns for placing the radiation source **95** in the exact position needed.

The thickness of lead box is preferably in the range of 0.5-1.0 cm, such that no radiation escapes therefrom. A window **96w** is provided in one of the walls of lead box **97** through which the radiation source **95** is passed into the patient's body. Window **96w** is covered with a lead door **96** having wall thickness similar to that of lead box **97**. Lead door **96** is removed before the radiation source **95** is sent out of lead box **97.**

The rod **91** holding the Tm170 source can be rigid so that it moves back and forth along a straight line only. Alternatively, the rod **91** holding the Tm170 source is made flexible such that it can be guided through curved passages to locations within the patient body which are not exactly in front of the window **96w**.

### Examples

### Example 1

### Preparation of a titanium capsule comprising thulium 170 and gold

Thulium 169 wire of 0.6 mm diameter was cut to obtain a section having a desired length (in the range between 1.0 mm and 5.0 mm). The thulium piece was then wrapped with a gold foil having thickness of about 0.1mm (Goodfellow, England). One end of a titanium tube (0.8 mm outer diameter, 0.7 inner diameter) was closed with a titanium plug. The plug is made of a titanium wire of diameter 0.7 mm (the inner diameter of the tube). A slice of 0.5 mm long of this wire was plugged into the titanium tube on one end and welded with a laser beam. The length of the titanium tube used depends on the length of the thulium piece + 0.5 mm for a plug insertion on each end.

The gold coated thulium was inserted into the titanium tube through the open end. The second end of the tube was also plugged and sealed by welding. The titanium tube is now ready to be activated in order to convert the Tm-169 into Tm-170 (the high flux reactor in Peten, the Netherlands). The Activation time and flux level, which determine the source activity, were described hereinabove.

### Example 2

### Preparation of a brachytherapy device, comprising thulium 170 and gold within a titanium shell attached to a guidewire (the embodiment shown in Figure 2)

A thulium wire of 0.6 mm diameter was cut to obtain a section having a desired length (in the range between 1.0 mm and 5.0 mm). The thulium piece was then wrapped with a gold foil having thickness of 0.1mm (Goodfellow, England).

One end of a titanium tube (0.8 mm outer diameter, 0.7 inner diameter) was plugged with a titanium plug. The plug is made of a titanium wire of diameter 0.7 mm (the inner diameter of the tube). A slice of 0.5 mm long of this wire was plugged into the titanium tube on one end and welded with a laser beam. The length of the titanium tube used depends on the length of the thulium piece + 0.5 mm for a plug insertion on one end and 2mm (or more) for inserting the flexible steel cable on the other end.

The gold coated thulium section was inserted into the titanium tube through the open end. The assembly was shipped to a nuclear reactor for neutron activation (the high flux reactor in Peten, the Netherlands). The Activation time and flux level, which determine the source activity, were described hereinabove.

After neutron activation, the thulium is radioactive and should be handled with the required care, and safety measures should be taken. The preparation of the source was completed in a hot cell or behind sufficient shielding. The steel wire was inserted into the open end of the titanium tube, using an epoxy glue and mechanical pressure to ensure a safe connection.

### Example 3

### Preparation of brachytherapy device comprising the capsule of the present invention (the embodiment shown in Figure 4)

A needle commonly applied in brachytherapy to implant radioactive seeds into patients was used to house the titanium capsule of Example 1, according to the following procedure (these needles have an inner diameter of 1.1 mm, outer diameter of 1.3 mm and are 200 mm long). The sharp end of the needle is removed and this end of the needle is carefully sealed by welding with copper or silver (care must be taken to keep the outer diameter of the needle as it is when seal-welding the end, otherwise it might not fit into the catheter used for delivering the source into the patient). The capsule of Example 1 is placed inside the needle through the open end. It is pushed all the way to the distal, sealed end using the inner part of the needle. This is a steel rod 1.0 mm diameter, 200 mm long, which is used to retain the capsule in place inside the needle. The other end of the needle, with the steel rod inside, is now welded to obtain a sealed source on both ends.

### Example 4

The following example describes an experiment which demonstrates the efficiency of the Tm-170+Au beta-gamma radiation source of the invention.

A Tm-170 radiation source for HDR having a gold coating was produced by irradiation of 13 mg natural thulium (Tm-169) rod, 5 mm long, 0.6 mm diameter, at the nuclear reactor in Peten, The Netherlands. The irradiation lasted 21 days at a neutron flux of 2.5x10**14 n/cm**2sec. The activity of the Tm-170 obtained was 5.4 curie. The rod was contained in a titanium capsule of 0.8 mm o.d., 0.7 mm i.d., 7 mm long. For the production of a usable source the capsule was sealed in a stainless steel needle, 20 cm long. 1.3 mm o.d, welded on both ends. The needle was then wrapped, at the source (distal) end, with a 0.1 mm thick gold foil. The Tm-170 source was kept in place inside the needle by a stainless steel rod inserted into the needle before welding the proximal end.

17 tumor bearing white mice were irradiated using the source described above. The mice were divided into two groups, 9 were irradiated with the gold foil wrapping the source, and 8 without the gold foil. Each of these two groups was divided into 3 irradiating time groups: 5 minutes, 10 minutes and 15 minutes. The mice were placed in a lead box of 3 mm wall thickness to protect their body from the radiation. A hole was made in the box through which the tumor was pushed out, for irradiation. A 2 mm o.d, 1.5 mm i.d, 15 cm long needle was inserted into the tumor to serve as a guide for the source. The experiment was done behind a thick lead-glass shielding with lead shielding on all other sides. The source was pushed into the guiding needle and was kept at the tumor for time length as mentioned above.

The tumor volume was measured every day before and after the treatment. In all mice the tumor growth stopped for days following the irradiation, or the tumor size was reduced. In order to eliminate the tumor completely, a second irradiation was needed about a week after the first one.

The experiment above shows that Tm-170 in combination with a gold coating is equally effective in treating cancer in comparison with non-coated Tm-170, despite the reduced beta emission. The gold coated Tm-170 has the advantage of having more photons absorbed in the tumor and is hence likely to be particularly useful in treating large tumors.

All of the abovementioned parameters are given by way of example only, and may be changed in accordance with the differing requirements of the various embodiments of the present invention. Thus, the abovementioned parameters should not be construed as limiting the scope of the present invention in any way. In addition, it is to be appreciated that the different cylindrical members, rods, wires, tubes, and other members, described hereinabove may be constructed in different shapes (e.g. having oval, square etc. form in plan view) and sizes differing from those exemplified in the preceding description.

## Claims

1. A capsule (**66**) for high dose rate brachytherapy, wherein the capsule comprises within its interior space thulium-170 (**64**) and further comprises at least one layer of a radiation emission modifying metal (**63**), wherein said layer is provided either internally within the capsule or on the outer surface thereof, wherein the activity of the radiation source within the capsule is not less than 3 curie, and wherein the capsule is made of titanium and the radiation emission modifying metal is provided as a gold coating or a platinum coating.

2. A capsule according to claim 1, wherein the thulium 170 is in the form of a cylindrical body and a gold coating is provided on the surface of said thulium 170, such that said coating is interposed between said thulium 170 and the walls of the capsule.

3. A capsule according to claim 1, wherein the radiation emission profile of the source placed therein is **characterized in that**:
(i) the level of the beta radiation emitted is reduced in comparison with that generated by thulium 170;
(ii) the photon emission spectrum of the source displays a lower ratio between the intensities of the characteristic peaks at 84 keV and 52 keV energy levels, relative to the ratio observed in the corresponding spectrum of thulium 170;
(iii) the photon emission spectrum of said source exhibits one or more peaks in the region between 65 keV and 88 keV energy levels indicative of photons emitted by the layer(s) of the radiation emission modifying metal;
(iv) the photon emission spectrum of said source comprises in addition to the discrete peaks also a continuous region
indicative of the emission of braking energy associated with the layer(s) of the radiation emission modifying metal.

4. A high dose rate brachytherapy device comprising an elongated, sealed tubular body (**57**) having a distal portion (**67**) and a proximal portion, wherein the capsule (**66**) according to claim 1 is placed in said distal portion (**67**) of said tubular body (**57**) and is affixed to said position by means of a rod (**11**) extending from said proximal end of said tubular body to said capsule.

5. A high dose rate brachytherapy device (**10**), comprising:
a) a thulium-170 radioactive section (**14**) attached to one end of a guiding wire (**11**), said radioactive section and a portion of said guiding wire being placed within the interior of a shell (**12**) and affixed to the walls thereof, with the proximal end of the guiding wire extending beyond the end of said shell; and
b) a radiation emission modifying metal (**13**), provided either internally within the shell or on the outer surface thereof, wherein the radiation source has an activity of not less than 3 curie, said metal being either gold or platinum.

6. A high dose rate brachytherapy device according to claim 5, wherein the radiation emission modifying metal is provided in the form of a gold coating or a platinum coating onto the radioactive section, such that said coating is interposed between said radioactive section and the shell.

7. A high dose rate brachytherapy device according to claim 5 or 6, wherein the radiation emission profile of the source placed therein is **characterized in that**:
(i) the level of beta radiation emitted therefrom is reduced in comparison with that generated by Tm-170;
(ii) the photon emission spectrum of said source displays a lower ratio between the intensities of the characteristic peaks at 84 keV and 52 keV energy levels, relative to the ratio observed in the corresponding spectrum of Tm-170;
(iii) the photon emission spectrum of said source exhibits one or more peaks in the region between 65 keV and 88 keV energy levels indicative of photons emitted by the layer(s) of the radiation emission modifying metal;
(iv) the photon emission spectrum of said source comprises in addition to the discrete peaks also a continuous region indicative of the emission of braking energy associated with the layer(s) of the radiation emission modifying metal.

8. A capsule according to claim 1, wherein the source has an activity in the range of 3-30 curie.

9. A brachytherapy device according to claim 4 or 6, wherein the source has an activity in the range of 3 to 30 curie.

## Patentansprüche

1. Kapsel (66) für hochdosierte Brachytherapie, wobei die Kapsel in ihrem Innenraum Thulium-170 (64) aufweist und ferner wenigstens eine Schicht aus einem Strahlungsemission modifizierenden Metall (63) aufweist, wobei die genannte Schicht entweder im Inneren der Kapsel oder an ihrer Außenfläche bereitgestellt ist, wobei die Aktivität der Strahlungsquelle in der Kapsel mindestens 3 Curie beträgt und wobei die Kapsel aus Titan hergestellt ist und das Strahlungsemission modifizierende Metall als eine Goldbeschichtung oder eine Platinbeschichtung bereitgestellt ist.

2. Kapsel nach Anspruch 1, wobei das Thulium-170 die Form eines zylindrischen Körpers hat und auf der Oberfläche des genannten Thulium-170 eine Goldbeschichtung bereitgestellt ist, so dass die genannte Beschichtung zwischen dem genannten Thulium-170 und den Wänden der Kapsel liegt.

3. Kapsel nach Anspruch 1, wobei das Strahlungsemissionsprofil der in sie gelegten Quelle **dadurch gekennzeichnet ist, dass**:
(i) der Pegel der emittierten Betastrahlung im Vergleich mit dem von Thulium 170 erzeugten geringer ist,
(ii) das Photonenemissionsspektrum der Quelle relativ zu dem Verhältnis, das in dem entsprechenden Spektrum von Thulium 170 zu beobachten ist, ein niedrigeres Verhältnis zwischen den Intensitäten der charakteristischen Spitzen bei Energiepegeln von 84 keV und 52 keV aufweist,
(iii) das Photonenemissionsspektrum der genannten Quelle eine oder mehrere Spitzen in dem Bereich zwischen Energiepegeln von 65 keV und 88 keV aufweist, die ein Indiaktor für von der/den Schicht(en) des Strahlungsemission modifizierenden Metalls emittierte Photonen sind, und
(iv) das Photonenemissionsspektrum der genannten Quelle zusätzlich zu den diskreten Spitzen auch einen kontinuierlichen Bereich umfasst, der ein Indikator für die Emission von bremsender Energie ist, die mit der/den Schicht(en) des Strahlungsemission modifizierenden Metalls assoziiert ist.

4. Vorrichtung für hochdosierte Brachytherapie, die einen länglichen, abgedichteten röhrenförmigen Körper (57) mit einem distalen Teil (67) und einem proximalen Teil aufweist, wobei die Kapsel (66) nach Anspruch 1 in den genannten distalen Teil (67) des genannten röhrenförmigen Körpers (57) gelegt und mithilfe eines Stabs (11) an der genannten Position befestigt ist, der von dem genannten proximalen Ende des genannten röhrenförmigen Körpers zur genannten Kapsel verläuft.

5. Vorrichtung für hochdosierte Brachytherapie (10), die Folgendes aufweist:
a) einen radioaktiven Thulium-170-Abschnitt (14), der an einem Ende eines Führungsdrahtes (11) angebracht ist, wobei der genannte radioaktive Abschnitt und ein Teil des genannten Führungsdrahts im Inneren einer Hülse (12) positioniert ist und an ihren Wänden befestigt ist, wobei das proximale Ende des Führungsdrahts sich über das Ende der genannten Hülse hinaus erstreckt,
b) ein Strahlungsemission modifizierendes Metall (13), das entweder im Inneren der Hülse oder an ihrer Außenfläche bereitgestellt ist, wobei die Strahlungsquelle eine Aktivität von mindestens 3 Curie hat, wobei das genannte Metall Gold oder Platin ist.

6. Vorrichtung für hochdosierte Brachytherapie nach Anspruch 5, wobei das Strahlungsemission modifizierende Metall in der Form einer Goldbeschichtung oder einer Platinbeschichtung auf dem radioaktiven Abschnitt bereitgestellt ist, so dass die genannte Beschichtung zwischen dem genannten radioaktiven Abschnitt und der Hülse liegt.

7. Vorrichtung für hochdosierte Brachytherapie nach Anspruch 5 oder 6, wobei das Strahlungsemissionsprofil der in sie gelegten Quelle **dadurch gekennzeichnet ist, dass**:
(i) der Pegel der davon emittierten Betastrahlung im Vergleich mit dem von ¹⁷⁰Tm erzeugten geringer ist,
(ii) das Photonenemissionsspektrum der genannten Quelle relativ zu dem Verhältnis, das in dem entsprechenden Spektrum von ¹⁷⁰Tm zu beobachten ist, ein niedrigeres Verhältnis zwischen den Intensitäten der charakteristischen Spitzen bei Energiepegeln von 84 keV und 52 keV aufweist,
(iii) das Photonenemissionsspektrum der genannten Quelle eine oder mehrere Spitzen in dem Bereich zwischen Energiepegeln von 65 keV und 88 keV aufweist, die ein Indikator für von der/den Schicht(en) des Strahlungsemission modifizierenden Metalls emittierte Photonen sind, und
(iv) das Photonenemissionsspektrum der genannten Quelle zusätzlich zu den diskreten Spitzen auch einen kontinuierlichen Bereich umfasst, der ein Indikator für die Emission von bremsender Energie ist, die mit der/den Schicht(en) des Strahlungsemission modifizierenden Metalls assoziiert ist.

8. Kapsel nach Anspruch 1, wobei die Quelle eine Aktivität im Bereich von 3 bis 30 Curie hat.

9. Brachytherapievorrichtung nach Anspruch 4 oder 6, wobei die Quelle eine Aktivität im Bereich von 3 bis 30 Curie hat.

## Revendications

1. Capsule (66) pour brachythérapie à dose élevée, la capsule comprenant dans son espace intérieur du thulium 170 (64) et comprenant en outre au moins une couche de métal de modification d'émission de rayonnement (63), ladite couche étant fournie soit intérieurement dans la capsule, soit sur la surface externe de celle-ci, l'activité de la source de rayonnement dans la capsule n'étant pas inférieure à 3 curies, et la capsule étant réalisée en titane et le métal de modification d'émission de rayonnement étant fourni sous forme de revêtement d'or ou de revêtement de platine.

2. Capsule selon la revendication 1, dans laquelle le thulium 170 a la forme d'un corps cylindrique et un revêtement d'or est fourni sur la surface dudit thulium 170, de telle sorte que ledit revêtement soit interposé entre ledit thulium 170 et les parois de la capsule.

3. Capsule selon la revendication 1, dans laquelle le profil d'émission de rayonnement de la source qui y est placée est **caractérisé en ce que** :
(i) le niveau du rayonnement bêta émis est réduit en comparaison à celui généré par le thulium 170 ;
(ii) le spectre d'émission photonique de la source affiche un rapport inférieur entre les intensités des pics caractéristiques aux niveaux d'énergie de 84 keV et 52 keV, par rapport au rapport observé dans le spectre correspondant du thulium 170 ;
(iii) le spectre d'émission photonique de ladite source présente un ou plusieurs pics dans la région entre les niveaux d'énergie de 65 keV et 88 keV indicatifs de photons émis par la ou les couches du métal de modification d'émission de rayonnement ;
(iv) le spectre d'émission photonique de ladite source comprend aussi en plus des pics discrets une région continue indicative de l'émission d'énergie de freinage associée à la ou aux couches du métal de modification d'émission de rayonnement.

4. Dispositif de brachythérapie à dose élevée comprenant un corps tubulaire allongé scellé (57) ayant une partie distale (67) et une partie proximale, dans lequel la capsule (66) selon la revendication 1 est placée dans ladite partie distale (67) dudit corps tubulaire (57) et est fixée à ladite position au moyen d'une tige (11) s'étendant depuis ladite extrémité proximale dudit corps tubulaire jusqu'à ladite capsule.

5. Dispositif de brachythérapie à dose élevée (10), comprenant :
a) une section radioactive au thulium 170 (14) attachée à une extrémité d'un fil de guidage (11), ladite section radioactive et une partie dudit fil de guidage étant placées à l'intérieur d'une coquille (12) et fixées aux parois de celle-ci, l'extrémité proximale du fil de guidage s'étendant au-delà de l'extrémité de ladite coquille ; et
b) un métal de modification d'émission de rayonnement (13), fourni soit intérieurement dans la capsule, soit sur la surface externe de celle-ci, la source de rayonnement ayant une activité de pas moins de 3 curies, ledit métal étant de l'or ou du platine.

6. Dispositif de brachythérapie à dose élevée selon la revendication 5, dans lequel le métal de modification d'émission de rayonnement est fourni sous forme de revêtement d'or ou de revêtement de platine sur la section radioactive, de telle sorte que ledit revêtement soit interposé entre ladite section radioactive et la coquille.

7. Dispositif de brachythérapie à dose élevée selon la revendication 5 ou 6, dans lequel le profil d'émission de rayonnement de la source qui y est placée est **caractérisé en ce que** :
(i) le niveau de rayonnement bêta émis par celui-ci est réduit en comparaison à celui généré par le Tm 170 ;
(ii) le spectre d'émission photonique de ladite source affiche un rapport inférieur entre les intensités des pics caractéristiques aux niveaux d'énergie de 84 keV et 52 keV, par rapport au rapport observé dans le spectre correspondant du Tm 170 ;
(iii) le spectre d'émission photonique de ladite source présente un ou plusieurs pics dans la région entre les niveaux d'énergie de 65 keV et 88 keV indicatifs de photons émis par la ou les couches du métal de modification d'émission de rayonnement ;
(iv) le spectre d'émission photonique de ladite source comprend aussi en plus des pics discrets une région continue indicative de l'émission d'énergie de freinage associée à la ou aux couches du métal de modification d'émission de rayonnement.

8. Capsule selon la revendication 1, dans laquelle la source a une activité dans la plage de 3 à 30 curies.

9. Dispositif de brachythérapie selon la revendication 4 ou 6, dans lequel la source a une activité dans la plage de 3 à 30 curies.
